# EUROPEAN PATENT APPLICATION

(11) **EP 3 896 697 A1**
(43) Date of publication of application: **20.10.2021**
(21) Application number: 20170185.1
(22) Date of filing: 17.04.2020
(51) Int. Cl.: G16B 20/30

(54) **METHOD AND DEVICE FOR IDENTIFYING MHC CLASS I-PRESENTED PEPTIDES FROM FRAGMENT ION MASS SPECTRA**

(71) Applicant: Julius-Maximilians-Universität Würzburg, 97070 Würzburg (DE)
(72) Inventor: Schilling, Bastian, 97078 Würzburg (DE); Schlosser, Andreas, 97076 Würzburg (DE); Erhard, Florian, 97073 Würzburg (DE)
(74) Representative: Lucke, Andreas

(57) **Abstract**

The present invention provides a method for determining MHC class I-presented peptides from fragment ion mass spectra, the method comprising:
- de novo peptide sequencing from the fragment ion mass spectra to generate candidate peptide sequences;
- determining, in a database, matching peptide sequences which match said candidate peptide sequences, wherein the database comprises target peptide sequences and decoy sequences;
- classifying the matching peptide sequences into classes of peptides, wherein each class refers to a functionally confined part of the genome;
- for each peptide length:
estimating a probability distribution of de novo peptide sequencing scores for false hits based on decoy hits, and estimating a probability of true hits based on subtracting of decoy hits from target hits, and
for each class: determining mixture coefficients of a mixture model of the true and false probability distributions; and

- filtering the matching peptide sequences using a statistical measure determined based on the deconvolution to determine the MHC class I-presented peptides.

## Description

### TECHNICAL FIELD

The present invention relates to a method for determining MHC class I-presented peptides from fragment ion mass spectra and a device for determining MHC class I-presented peptides from fragment ion mass spectra.

The present invention also relates to a computer-readable storage medium storing program code, the program code comprising instructions for carrying out such a method.

### BACKGROUND

Peptide presentation by MHC class I (MHC-I) is one of the main pillars of the adaptive immune system and plays a central role in cancer, infection and autoimmunity. Cytotoxic CD8+ T lymphocytes (CTLs) continuously scan these peptides on the surface of all nucleated cells with their T cell receptor. CTLs are able to recognize "non-self'-peptides (e.g. from a pathogen, a neoantigen due to mutation in tumor cells, or anything else that is not presented by a normal, healthy cell) and to subsequently elicit immune responses. The comprehensive identification of all peptides that are presented by MHC-I (the so-called immunopeptidome) therefore is of great interest. The central technique that has been established for this purpose is mass spectrometry (MS) after biochemical purification of MHC-I peptides (Freudenmann et al., 2018).

So far, common database search engines, such as Mascot (Perkins et al., 1999), Andromeda (Cox et al., 2011) or Sequest (Eng, Jimmy K., Ashley L. McCormack, 1994) have been applied for identifying MHC-I peptides from fragment ion mass spectra. All these tools use similar algorithms to calculate a matching score between experimental and theoretical fragment ion mass spectra. These search engines have been developed and optimized for classical proteomics analyses, i.e. identification of tryptic peptides. Since MHC-I peptides are generated by the proteasome without stringent cleavage specificity, the search space for MHC-I peptides is substantially larger. Thus, when applied for the identification of MHC-I peptides common search engines provide only limited sensitivity, i.e. they fail to identify a significant percentage of MHC-I peptides.

De novo sequencing is an alternative approach for peptide identification from fragment ion mass spectra (Muth et al., 2018). In contrast to classical database searching, de novo sequencing algorithms infer the peptide sequence directly from the fragment ion mass spectra without utilizing a sequence database. Subsequently, peptides can be compared to sequence databases to identify the protein they originate from (Kim et al., 2009). De novo sequencing is more unbiased than database searching and can identify peptides not present in common databases. However, high quality fragment ion mass spectra are required for reliable sequence determination by de novo sequencing. In addition, concepts for statistical filtering to control false discovery rates (FDRs) are largely missing for de novo sequencing.

Gene expression studies (e.g. TCGA) uncovered various forms of aberrant transcription in cancer cells including retained introns, neo-junctions and neo-transcripts from intergenic regions. Moreover, dysregulation of the translational machinery has been shown to be prevalent in tumor cells (Sendoel et al., 2017). This led to the hypothesis that all these mechanisms result in the translation of aberrant open reading frames (ORFs) and that these represent an important source for tumor-specific MHC-I epitopes.

Erhard et al., 2018 and others (Laumont et al., 2016) previously combined MS with next generation sequencing (NGS; ribosome profiling and RNA-seq, respectively), and provided first evidence for so-called cryptic peptides (that are encoded outside the known proteome) in immunopeptidomes. These approaches were based on compiling experiment-specific target sequence databases from the NGS data to screen the MS data. These approaches have several disadvantages and so far only resulted in the identification of a small number of cryptic peptides (126 in the Laumont et al. study, 132 in Erhard et al.).

### SUMMARY OF THE INVENTION

The objective of the present invention is to provide a method for determining MHC class I-presented peptides from fragment ion mass spectra and a device for determining MHC class I-presented peptides from fragment ion mass spectra, which overcome one or more of the above-mentioned problems of the prior art.

A first aspect of the invention provides a method for determining MHC class I-presented peptides from fragment ion mass spectra, the method comprising:
- de novo peptide sequencing of the fragment ion mass spectra to generate candidate peptide sequences;
- determining, in a database, matching peptide sequences which match said candidate peptide sequences, wherein the database comprises target peptide sequences and decoy sequences;
- classifying the matching peptide sequences into classes of peptides, wherein each class refers to a functionally confined part of the genome;
- for each peptide length:
   estimating a probability distribution of de novo peptide sequencing scores for false hits based on decoy hits, and estimating a probability distribution of true hits based on subtracting of decoy hits from target hits, and
   for each class: determining mixture coefficients of a mixture model of the true and false probability distributions; and
- filtering the matching peptide sequences using a statistical measure determined based on the mixture model to determine the MHC class I-presented peptides.

The fragment ion mass spectra can be from a sample of a subject. The method can comprise an initial step of obtaining the fragment ion mass spectra from the sample.

Typically, the fragment ion mass spectra originate from a subject sample. The subject sample may be a tissue sample such as tumor tissue, healthy tissue or blood. In other embodiments, the fragment ion mass spectra can be determined from other sources such as cell lines or animals.

Preferably, the target sequences comprise amino acid sequences of the genome of the corresponding species of the subject.

The candidate peptide sequence can be for example a predetermined number of candidate peptide sequences.

Determining the probability distributions is performed separately for each peptide length because the overall score distributions for distinct peptide lengths differ.

Estimating a probability distribution of de novo peptide sequencing scores for false hits based on decoy hits can be performed for example as follows:
The measured false hits are binned in a histogram according to their de novo peptide sequencing score. Using a mathematical model, e.g. a non-parametric regression model, a curve is fitted to the histogram. This fitted curve is then used as the estimate of the false probability distribution.

Similarly, estimating a probability distribution of true hits based on subtracting of decoy hits from target hits can be performed as follows: The decoy hits are binned into a histogram, wherein individual bins correspond to ranges of de novo peptide sequencing scores. In the same way, target hits are binned into a histogram, wherein individual bins correspond to ranges of de novo peptide sequencing scores. The decoy histogram is subtracted from the target histogram and a mathematical model, e.g. a non-parametric regression model, is used to fit a curve to the difference histogram.

Estimating the true probability distribution can be performed based on target and decoy hits that are from a specific class. Preferably, this can be the class of CDS peptides. This is advantageous because the CDS peptides comprise only few false hits. Hence, the CDS peptide target hits are a good estimate of the true hits. Hence, the CDS peptide hits are particularly suitable for estimating the true distribution. It can be assumed that the true and false hits in different classes follow the same distribution (with merely different mixture coefficients), hence, the distributions of one or more selected classes, e.g. the CDS peptides, can be used for estimating the true distribution for all classes.

Determining mixture coefficients of a mixture model of the true and false probability distributions can be performed by using expectation maximization algorithms. The mixture coefficients are preferably determined separately for each class because the mixture coefficients are expected to be different for different classes.

The method of the first aspect is tailored to determining MHC-I peptides. Embodiments of the method of the first aspect are based on de novo peptide sequencing, efficient sequence search in ultra-large databases, and provide a novel way to assess the statistical significance of identifications and to control the peptide-wise FDR.

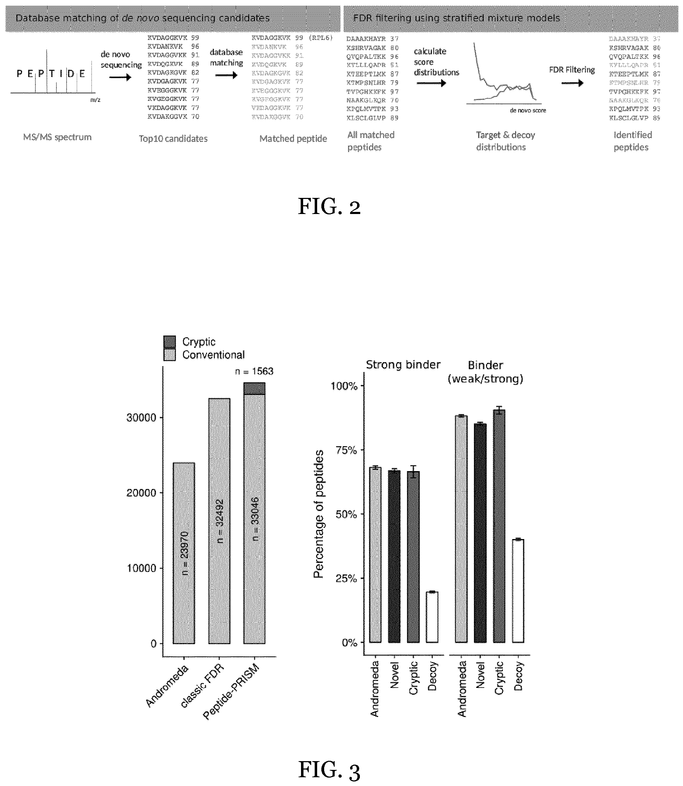

De novo sequencing infers peptide sequences directly from the fragment ion mass spectrum. Many approaches for de novo peptide sequencing have been developed (see References in (Muth et al., 2018)), but are generally considered inferior to sequence database search (Muth et al., 2018). The method of the first aspect makes use of the discovery that for high mass accuracy fragment ion spectra that are produced by modern mass spectrometers, such as Orbitrap or Q-TOF type instruments, and due to the length restriction of MHC-I peptides (mostly 8-13 amino acids), de novo peptide sequencing is much more sensitive (>35% more identified peptides) when the FDR is controlled at 1% (see FIG. 2).

In a first implementation of the method according to the first aspect, the fragment ion mass spectra originate from a sample of a subject and the target peptide sequences are amino acid sequences of the species of the subject, wherein in particular the target peptide sequences comprise peptide sequences corresponding to the 6-frame translated genome and/or 3-frame translated transcriptome.

The amino acid sequences of the species of the subject may be obtained from a database, e.g. from a published database of amino acid sequences of the species (e.g. humans).

In a further implementation, the target sequences comprise variant target sequences, such as generated due to mutations from DNA sequences, proteasome-catalyzed peptide splicing, all possible single amino acid substitutions (from annotated proteins) and/or frameshift peptides. This has the effect that also variants of the target sequences can be captured. For example, a peptide sequence can be determined even if a mutation of a DNA sequence has occurred.

Preferably, the decoy sequences comprise inverted sequences. All but a negligible number of those sequences are non-biological and therefore represent by definition false hits if identified in the search. As inverting the sequences preserves all relevant physico-chemical properties of the peptides, the probability of a false hit in the decoy database is equal to a false hit in the target database. This is a necessary condition for estimating false discovery rates.

In a further implementation, the database matching comprises using the Aho-Corasick algorithm to scan said target sequences and optionally said variants of said target sequences, in addition to scanning all considered sequences in their inverted form. Experiments have shown that the Aho-Corasick algorithm is extremely efficient in scanning the sequences.

In a further implementation, the classifying of the matching peptide sequences comprises prioritizing and/or filtering hits and/or reducing to unique sequences.

Prioritization in general has the advantage that the statistical power of identifying peptides in higher-priority classes does not suffer from the larger search spaces of lower-priority classes.

For example, the prioritization can involve assigning one or more of the following priorities: CDS > 5'-UTR > Off-Frame > Frameshift > 3'-UTR > ncRNA >Substitution >Intronic > Intergenic > PCPS.

These specific prioritizations are particularly useful because higher-priority classes are smaller and contain more bona-fide MHC-I presented peptides.

Preferably, the prioritization is performed by successively searching within all sequences belonging to two or more of the classes CDS, 5'-UTR, off-frame, 3'-UTR, ncRNA, intronic, and intergenic.

In a further implementation, the fragment ion mass spectra are from a subject sample, which is derived from a patient, in particular a cancer patient. The presented method has shown particularly useful in oncology.

In a further implementation, the top n sequence candidates of each fragment ion mass spectrum are used for database matching, wherein n is an integer between 5 and 200, preferably between 7 and 20. In particular, the top 10 sequence candidates can be used. This has shown to give a good balance between considering sufficient potential candidates, but still avoiding too many search for unlikely matches.

In a further implementation, determining the matching peptide sequences comprises adding isobaric amino acid substitutions (L -> I), the amino acid variants N -> D within the sequence motif NG, and the amino acid variants Q -> E within sequence candidates with N-terminal pyro-glutamate to the Aho-Corasick keyword trie for all candidate sequences.

In a further implementation, the classes comprise at least one of:
CDS (in-frame with annotated protein),
5'-UTR (contained in annotated mRNA, consistent with its introns, overlapping with 5'-UTR),
off-frame (off-frame contained in the coding sequence, consistent with its introns),
3'-UTR (all others that are intron-consistently contained in an mRNA),
ncRNA (intron-consistently contained in an annotated ncRNA),
intronic (intersecting any annotated intron),
   or
intergenic.

In a further implementation, the fragment ion mass spectra have been obtained using collision-induced dissociation (CID), higher-energy C-trap dissociation (HCD), electron transfer dissociation (ETD) or combinations of these fragmentation techniques.

A second aspect of the invention refers to a device for determining MHC class I-presented peptides from fragment ion mass spectra, wherein the device is configured to carry out the method of one of the previous implementations.

A further aspect of the invention refers to a computer-readable storage medium storing program code, the program code comprising instructions that when executed by a processor carry out the method of the second aspect or one of the implementations of the second aspect.

### BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.
- FIG. 1: is a flow chart illustrating a method for determining MHC class I-presented peptides from fragment ion mass spectra in accordance with an embodiment of the present invention,
- FIG. 2: is a schematic overview of the a method in accordance with an embodiment of the present invention,
- FIG. 3: shows diagrams comparing the number of peptide sequences identified with different methods,
- FIG. 4: shows several diagrams for illustrating the fitting of the score distributions,
- FIG. 5: shows several score distributions as obtained with a method in accordance with an embodiment of the present invention, and
- FIG. 6: shows several score distributions as obtained with a method in accordance with an embodiment of the present invention, wherein the databases have been stratified.

### DETAILED DESCRIPTION OF EMBODIMENTS

The foregoing descriptions are only implementation manners of the present invention, the scope of the present invention is not limited to this. Any variations or replacements can be easily made through a person skilled in the art. Therefore, the protection scope of the present invention should be subject to the protection scope of the attached claims.

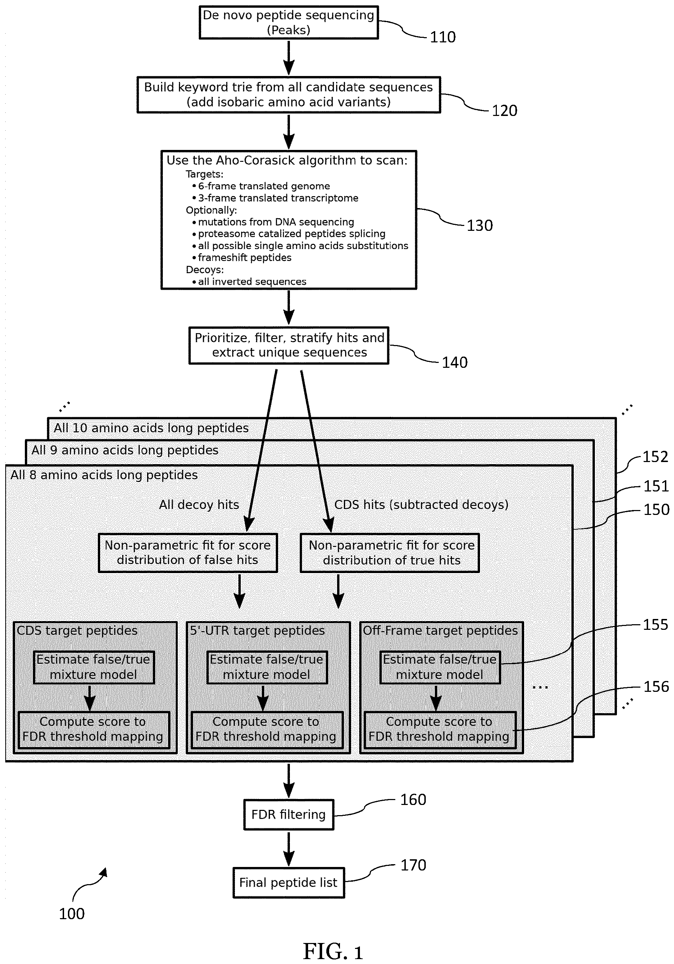

FIG. 1 shows a method 100 for determining MHC class I-presented peptides from fragment ion mass spectra. The method comprises a first step 110 of performing de novo peptide sequencing from the fragment ion mass spectra. Different software tools are available for de novo peptide sequencing. For example, a commercial software tool (PEAKS Studio, BSI, Canada) can be applied. Other software tools can be used as well. The fragment ion mass spectra may be e.g. from a dataset, e.g. a published dataset, or they may be from a sample of a subject.

In a second step 120, a keyword trie from all candidate sequences is built.

In a third step 130, the Aho-Corasick algorithm is used to scan for targets. In particular, the 6-frame translated and/or 3-frame translated transcriptome is searched. Optionally, also mutations from DNA sequencing, proteasome catalyzed peptide splicing, all possible single amino acids substitutions and/or frameshift peptides are searched. As decoys, all inverted sequences are used.

It is understood that in other embodiments of the method, other search algorithms may be used.

In a fourth step 140, prioritizing, filtering and stratifying of hits is performed and unique sequences are extracted.

Subsequently, separate processing is performed for peptide sequences of different lengths. For example, peptide sequences of lengths of 8 to 13 amino acids are processed separately for each length.

In step 150, only peptide sequences that are 8 amino acids long are processed. This processing involves that for all decoy hits, a non-parametric fit for the score distribution of false hits is performed. Additionally, a non-parametric fit for the score distribution of true hits is performed.

Subsequently, for CDS target peptide sequences, for 5'-UTR target peptide sequences and for off-frame target peptide sequences (as well as possibly other classes), a false/true mixture model is estimated in step 155, which thus allows computing a score to FDR threshold mapping.

Processing for the peptide sequences of other lengths is separately performed in steps 151 and 152.

After the FDR thresholds have thus been determined separately for different lengths and for different classes of peptides, FDR filtering is performed in step 160, to generate the final peptide sequence list, which can be output in step 170.

*De novo* sequencing infers peptide sequences directly from the fragment ion mass spectrum. Many approaches for *de novo* peptide sequencing have been developed (see References in (Muth *et al.,* 2018)), but are generally considered inferior to sequence database search (Muth *et al.,* 2018). For high mass accuracy fragment ion spectra that are produced by modern mass spectrometers, such as Orbitrap or Q-TOF type instruments, and due to the length restriction of MHC-I peptides (mostly 8-13 amino acids), *de novo* peptide sequencing is much more sensitive (>35% more identified peptides) when the FDR is controlled at 1% (see FIG. 3).

The presented method uses *de novo* peptide sequencing (applying for example the software tool PEAKS Studio, (Ma *et al.,* 2003)) to generate a number of sequence candidates, e.g. ten candidates, for each fragment ion spectrum. To identify their potential origin in the genome, the Aho-Corasick algorithm is adopted to simultaneously search for the millions of candidate sequences derived from a typical immunopeptidome data set in the 6-frame translated genome and 3-frame translated transcriptome sequence with great efficiency.

Of note, while both *de novo* peptide sequencing and the Aho-Corasick algorithm are well-known among experts for mass spectrometry and computer science, respectively, the presented method combines them to rapidly search ultra-large databases for MHC-I peptide candidates. The combination of *de novo* peptide sequencing and database search essentially constitutes a new sequence database search approach. It is important to note that it is orders of magnitudes faster than any existing approach for ultra-large databases due to performing the search on the level of sequences (instead of fragment ion spectra) using the Aho-Corasick algorithm.

This, in principle, enables the presented method to employ the standard target-decoy database approach (TDA) (Elias *et al.,* 2007) to estimate and control FDRs. However, the statistical power of TDA is highly limited when the search space is large. Thus, the presented TDA-based approach is implemented with much greater sensitivity. The search space is stratified into biologically motivated categories, which may include one or more of CDS (in-frame with annotated protein), 5'-UTR (contained in annotated mRNA, consistent with its introns, overlapping with 5'-UTR), off-frame (off-frame contained in the coding sequence, consistent with its introns), 3'-UTR (all others that are intron-consistently contained in an mRNA), ncRNA (intron-consistently contained in an annotated ncRNA), intronic (intersecting any annotated intron), intergenic (anything else found in the genome). For each fragment ion mass spectrum, it is first tested whether any of the ten candidates matches to a CDS sequence. If this is not the case, subsequently the other categories as defined above are considered.

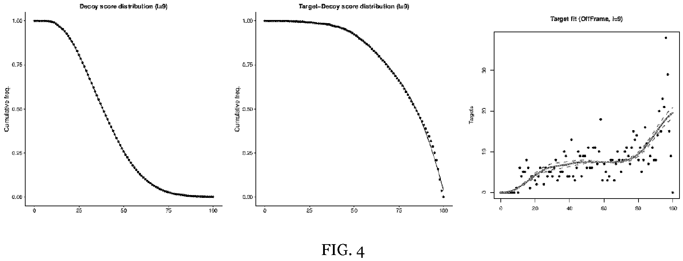

The result at this point comprises sets of target and decoy peptides for each category with associated *de novo* score. It is assumed that both true sequences and false sequence are characterized by a certain (unknown) *de novo* score distribution, respectively. As the *de novo* score only depends on the quality of the spectrum, but not on the size of the category, it can be assumed that the *true* as well as the *false distribution* are the same across all categories (but distinct for each sequence length). Unimodal penalized B-spline regression can be used to fit both distributions in a non-parametric manner. All decoys of a certain sequence length can be used for the *false distribution* and the decoy subtracted histogram of CDS peptides of a certain sequence length to fit the *true distribution* (see FIG. 4).

FIG. 2 shows an overview of a further presented method. Mass spectra are analyzed using de novo sequencing, to determine a top number of candidates. Subsequently, database matching is performed to determine matches in a predetermined database. Then, shown on the right side in FIG. 2, FDR filtering is performed using stratified mixture models. Therein, a de novo score for target and decoy distributions is determined. Based on a comparison of the distributions, a confidence measure, e.g. a threshold value, is determined. The threshold can be used for FDR filtering to identify the peptide sequences.

FIG. 3 shows on the left the number of peptides identified for sample MM15. The data for this sample are from Bassani-Sternberg M, Bräunlein E, Klar R, Engleitner T, Sinitcyn P, Audehm S, et al. "Direct identification of clinically relevant neoepitopes presented on native human melanoma tissue by mass spectrometry". Nat Commun. 2016;7:13404. Andromeda is the sequence database search approach used previously for these data and represents the originally published numbers, classic FDR and Peptide-PRISM are described in the methods. On the right statistics on predicted MHC-I binding is shown. Novel and cryptic peptides consist of the same percentage of netMHCpan 4.0 predicted MHC-I binders as the published peptide set. Novel peptides are proteome-derived peptides identified by our approach but not in the original report. Error bars represent 95% binomial confidence intervals.

FIG. 4 illustrates the fitting of the score distributions. On the left, the cumulative histogram is shown for decoy scores (sequence length 9, dots), together with the fitted distribution (line). In the middle, the same is shown for target scores. On the right, the histogram of "off-frame" target peptides of length 9 is shown (dots) together with the fitted mixture model.

Then, for each sequence length and category, the mixture model consisting of the corresponding false and true distributions to the *de novo* scores of all corresponding target peptides is fitted. From this mixture distribution, any measure of statistical confidence (such as FDRs) can be extracted.

In the following, further embodiments are described in detail:
Peptide presentation by major histocompatibility complex class I (MHC-I) constitutes a main pillar of the adaptive immune system to fight infection and cancer. Mass spectrometric analysis of MHC-I immunopeptidomes allows the identification of thousands of T-cell epitopes (REF). However, current approaches are restricted to identify peptides in predefined databases. Here, *de novo* peptide sequencing, efficient sequence search in ultra-large databases (herein referred to as dbNovo) and a new statistical approach (herein also referred to as Peptide PRISM) are combined for reliable identification of T cell epitopes. We show that peptides originating for proteasome-catalyzed peptide splicing (PCPS) do not significantly contribute to MHC-I immunopeptidomes but rather represent computational and experimental artifacts. In contrast, we identified thousands of cryptic peptides constituting up to 15% of MHC-I tumor immunopeptidomes in an MHC-I allele specific manner. Cryptic peptides commonly mapped to the C-terminal end of open reading frames indicating they can directly enter the ER independently of processing by the proteasome. The findings reveal cryptic peptides representing a new class of candidate epitopes for novel immunotherapies.

Ribosome profiling (Ribo-seq) has provided compelling evidence for the translation of thousands of open reading frames (ORFs) outside of the annotated proteome. As such, more than half of all human genes encode so called upstream ORFs (uORFs), many of which are strongly translated. Additional cryptic ORFs originate from aberrant splicing events (e.g. intron retention) or are located in transcripts so far thought to be non-coding. Their products so far largely escape detection by whole proteome mass spectrometric (MS) approaches. MHC-I peptide presentation, is dependent on translation rates rather than on cellular protein abundance. Consequently, by analyzing MHC-I immunopeptidome data it could be determined that more than 2% of the peptides bound to MHC-I in human fibroblasts originate from cryptic, i.e. so far unknown ORFs identified by Ribo-seq.

The standard analysis workflow for mass spectrometry-based proteomics can be performed using database searching coupled to false discovery rate (FDR) estimation using the target-decoy approach. It is hypothesized that the length restriction of MHC-I peptides of predominantly 8-11 amino acids (aa) should enable *de novo* direct peptide sequencing based on state-of-the-art high mass accuracy tandem mass spectra. To test this, the presented method presents a computational framework for the analysis of MHC-I immunopeptidomes (as illustrated in FIG. 2) and performed *de novo* sequencing of MHC-I peptides from a tumor sample of a melanoma patient (MM15). Database matching of *de novo* sequencing candidates (dbNovo) resulted in ∼35% more, high-confident conventional peptides derived from proteins (1% FDR based on classic target-decoy approach) than based on the previously employed database searching approach (see FIG. 3 for a comparison). Of note, the newly identified peptides exhibited the same frequency of MHC-I binders as the previously identified peptides predicted by NetMHCpan 4.0.

To comprehensively identify cryptic MHC-I peptides, two major, previously not addressed issues have been solved: First, the presented method provides a highly efficient algorithm enabling to search for millions of peptides in sequence databases in the order of gigabases in less than an hour. Second, to achieve high sensitivity in large search spaces while controlling the FDR, mixture modeling is employed that effectively deconvolutes the overall *de novo* score distribution into components of false and true identifications. The peptide-PRISM approach identified 1,563 cryptic peptides at 1% FDR, corresponding to more than 4.5% of all identified peptides for MM15 (FIG. 3). The predicted binding affinity to MHC-I was indistinguishable from conventional peptides, and the median intensities of the identified cryptic peptides obtained from peak areas of extracted ion chromatograms did not show any difference compared to conventional peptides. 24 out of 24 identified cryptic peptides were successfully validated using MS of synthetic peptides. In summary this highlights stringent control of the FDR by the presented method.

The sequence database for matching the sequence candidates consisted of the 6-frame translated human genome, transcriptome from Ensembl (including all coding and non-coding splice variants), PCPS peptide database (normal and reverse *cis*-spliced peptides with a maximal intervening distance of 25), the human proteome with all possible substitutions of a single aa, and any peptide that could be generated from ribosomal frameshifting in the human proteome (for details see Methods). Peptides in this database can be stratified into nine categories, in addition to conventional peptides from coding sequences. Importantly, each of these strata has a distinct size and likelihood for identifying peptides, which has profound impact for estimating FDRs (see explanation below). Peptide-PRISM allows controlling the FDR per stratum as opposed to a global FDR control by the classic target-decoy approach. Moreover, to assess the relative composition of the different strata in the MHC-I immunopeptidome, Peptide-PRISM can estimate the number of peptides in each stratum without FDR filtering.

The recent success of cancer immunotherapies, such as immune checkpoint blockade or adoptive T cell transfer has demonstrated that T cells can indeed recognize and eliminate tumor cells *in vivo.* However, the tumor-specific antigens that are recognized by the corresponding cytotoxic T cells mediating the anti-tumor effect are generally unknown. Neoantigens recently came into the focus as a potentially relevant class of tumor-specific targets. However, despite tremendous efforts, identified neoantigens are scarce raising considerable doubt of whether these MHC-I peptides indeed represent a major class of tumor-specific antigens. PCPS peptides have also been proposed as potential tumor-specific antigens. Our analyses, however, exclude a significant contribution of PCPS peptides to the immuopeptidome *in vivo.* In contrast, cryptic peptides that have been discovered already more than 30 years ago have been largely neglected as potential tumor-specific targets so far. This is likely due to missing computational methods and tools for their efficient identification. Applying dbNovo and Peptide-PRISM, we identified cryptic peptides to be a substantial part of immunopeptidomes in different types of tumors. Thus, the presented method allows for functional analysis of cryptic peptides and will support the evaluation of those as potential new targets for cancer immunotherapy.

In a preferred embodiment of the presented method, de *novo* sequencing was performed with PEAKS X (Bioinformatics Solutions Inc., Canada). Raw data refinement was performed with the following settings: Merge Options: no merge, Precursor Options: corrected, Charge Options: no correction, Filter Options: no filter, Process: true, Default: true, Associate Chimera: yes. *De novo* sequencing was performed with Parent Mass Error Tolerance set to 15 ppm for dataset PXD004894 (melanoma) and with 10 ppm for all other data sets. Fragment Mass Error Tolerance was set to 0.015 Da, and Enzyme was set to none. The following variable modifications have been used: Oxidation (M), pyro-Glu from Q (N-term Q) and carbamidomethylation (C). A maximum of 3 variable PTMs were allowed per peptide. Up to 10 *de novo* sequencing candidates were reported for each identified feature. Two tables ("all de novo candidates" and "de novo peptides") were exported from PEAKS for further analysis.

### Sequence search

In a preferred embodiment of the presented method, to efficiently search ultra-large sequence databases, first a keyword trie is built from all *de novo* candidate sequences. To account for additional variable modifications (pyro-Glu from N-terminal Glu, deamidation at NG), and for the isobaric Leu and Ile, all possible combinations of the corresponding sequences were inserted into the trie for each *de novo* candidate. Then the Aho-Corasick algorithm is employed scanning through the 6-frame translation of the genome (here reference assembly HG38), the 3-frame translation of the transcriptome (here Ensembl 90). Optionally, on-the-fly generated PCPS peptides (normal and reverse *cis*-spliced, maximal intervening sequence length of 25 aa from all annotated proteins), all possible on-the-fly generated single aa substitutions from annotated proteins (i.e. at each position in each protein each of the 18 substitutions excluding Leu), and all possible, on-the-fly generated frameshift peptides (-3,-2,-1,1,2,3 nucleotide shifts at each position in each annotated protein) are scanned as well. In addition, all considered sequences are additionally reversed and scanned as well.

Preferably, all identified string matches from the 6-frame translated genome and 3-frame translated transcriptome are categorized into CDS (in-frame with annotated protein), 5'-UTR (contained in annotated mRNA, consistently with its introns, overlapping with 5'-UTR), Off-Frame (off-frame contained in the coding sequence, consistently with its introns), 3'-UTR (all others that are intron-consistently contained in an mRNA), ncRNA (intron-consistently contained in an annotated ncRNA), Intronic (intersecting any annotated intron) or Intergenic. Then, for each feature, the category with highest priority (CDS>5'-UTR>Off-Frame>Frameshift>3'-UTR> ncRNA>Substitution> Intronic> Intergenic>PCPS) was identified, and all other hits among the 10 *de novo* candidates were discarded.

Next, multiple features for the same sequence were identified and only the feature with maximal score among those all was retained. Finally, if the remaining candidate for a feature was *a*, the originally best candidate (irrespective whether it was found in the sequence database or not) was *f* and the next best distinct remaining candidate (i.e. in the same category, but a distinct sequence; isobaric Ile/Leu and additionally considered modifications were not treated as distinct here) was *n*, the feature was discarded if ALC(*a*)<ALC(*f*)-δ_{f} or ALC(*a*)<ALC(*n*)+δ. Here, we set δ_{f}=15 (the maximal difference to the originally top candidate), and δₙ=16 (the minimal difference to the next best candidate sequence from the same category).

This procedure results in a list of unique peptide sequences annotated with its best ALC score, its category and whether it is from the target or decoy database. In principle, after discarding all categories but CDS, a standard target-decoy approach can be utilized to filter proteome-derived peptides by false discovery rate (FDR).

### Statistical filtering

The preferred embodiment in therefore following referred to as Peptide-PRISM is built on the following mixture modeling approach: For a given target score distribution with density *f_{T}(x)* and decoy score distribution with density *f_{D}(x),* the overall score distribution of all remaining peptides of a given length *l* and category *c* is given by the density *f(x)*=*w_{l,c} f_{T}(x)*+*(1-w_{l,c})f_{D}(x).* Here, *w_{l,c}* is the total fraction of true targets with length *l* from category *c*. For the rationale of this model, see below.

Separately for each observed peptide length, category and target/decoy status, Peptide-PRISM first builds histograms of the (integer) ALC scores of the remaining unique peptides. Then, for each peptide length, first all decoy histograms are summed up to fit the decoy score distribution using unimodal penalized B-spline regression. The target score distribution is fit in the same manner after subtracting the CDS decoy histogram from the CDS target histogram.

Then, for each peptide length *l* and category *c*, *w_{l,c}* is estimated by maximum likelihood based on the respective target and decoy score histograms. The expected number of true and false targets per ALC score is computed from the mixture model. Finally, these expected numbers of true and false targets are then used to compute FDRs per peptide length and category.

### Putative ORF identification

Each remaining peptide has been identified to originate from a location on the genome, or potentially several locations if the same sequence occurs multiple times in its category (or any Ile, Leu / variable modification variant). For each location, all potential ORFs are identified (there can be more than one because all paths via exon-exon junctions of annotated transcript isoforms were considered here in addition to the path on the genome). Since translation might initiate at non-AUG start codons, two kinds of ORFs were considered here: for non-prioritized ORFs, along each path in the genome or transcriptome upstream of the peptide location, the closest in-frame start codon candidate was identified (one of AUG, CUG, GUG, ATC, ACG). For prioritized ORFs, first any closest in-frame AUG was identified. If none was found, the closest CUG was identified, then followed by GUG, ATC and ACG. From all remaining start codon candidates, the one closest to the peptide was chosen for both kinds of ORFs. The same procedure was repeated downstream of the peptide location to identify the stop codon (only non-prioritized). Finally, for peptides with more than one location, the location giving rise to the shortest prioritized ORF was chosen.

### Prediction of MHC-I peptide binding

NetMHCpan 4.0 was run on all finally remaining peptide sequences with the MHC-I alleles given in the original publication for the corresponding patient or cell line. The allele with the minimal rank reported by netMHCpan was annotated. For samples with unknown MHC-I genotype Gibbs Clustering was performed with GibbsCluster 2.0, and alleles were manually assigned by comparing motifs from clustering results with known MHC-I peptide motifs.

In the following, a preferred implementation of the target-decoy approach (TDA) to estimate false discovery rates (FDRs) for mass spectrometry (MS) experiments is presented, and it is shown how it can be employed in conjunction with *de novo* peptide sequencing. We then describe consequences of its use in stratified databases (e.g. composed of conventional and cryptic peptides). Finally, we discuss an example from the literature, where non-compliance with the fundamental conditions of TDA has led to contradictory results.

### 1. Target-decoy approach

TDA is the most widely (and virtually exclusively) used way to estimate FDRs for peptide identifications from MS data.

Preferred main components for TDA are (i) a target sequence database (ii) a decoy sequence database, and (iii) a program that, based on an input sequence database D, assigns a peptide from D and an associated score to each tandem mass spectrum (peptide-spectrum matches, PSMs).

Many PSMs refer to false peptides (i.e. peptides that were actually not in the sample analyzed by MS). Therefore, a threshold *t* is used, and all PSMs exceeding *t* are called discoveries. The purpose of TDA is to estimate the rate of false discoveries, i.e. the percentage of false PSMs in the set of all PSMs exceeding *t.* In addition to these so-called PSM FDRs, peptide FDRs can be defined as the percentage of false peptides in the set of all peptides from PSMs exceeding *t.*

The TDA is as follows: If all decoy peptides are false (**condition 1**), and the probability for a discovery from the decoy database is equal to the probability for a false discovery from the target database (**condition 2**), then the expected value of the number of false discoveries from the target database is the number of decoy discoveries. The FDR can then be computed by dividing the expected value by the total number of target discoveries.

Violations of condition **1** lead to conservative FDR estimates (i.e. the actual FDR is less than the estimated FDR). Condition 1 is usually ensured by filling the decoy database with peptides that are not in the known proteome.

More problematically, violations of condition 2 can lead to underestimated FDRs (i.e. the actual FDR is greater than the estimated FDR). This is the case if the probability of decoy discoveries is less than the probability of false target discoveries. Condition 2 holds if (i) target and decoy databases have the same size and (ii) the physicochemical properties of target and decoy peptides are similar. This is usually achieved by reversing or shuffling the target database.

Obviously, condition 2 is crucial. For the sake of the following arguments, we will reformulate it:

### Master condition:

The score histogram of decoys is the same as the score histogram of false targets.

There are two important remarks here: First, the "decoys" and "targets" might both refer either to PSMs or peptides. Second, if the master condition as stated is fulfilled, condition 2 above is fulfilled (as we defined "discoveries" by a threshold on the score). The converse is not necessarily true, as condition 2 demands equality only for the chosen threshold, not for all thresholds.

Moreover, the FDR as defined above is the expected value of a random variable and depends on the choice of the decoy database. It is therefore pertinent to make sure the estimate of the FDR is precise by ensuring a large enough decoy database.

Peptides in the databases can be categorized into three classes:
1. Decoy peptides
2. False target peptides
3. True target peptides

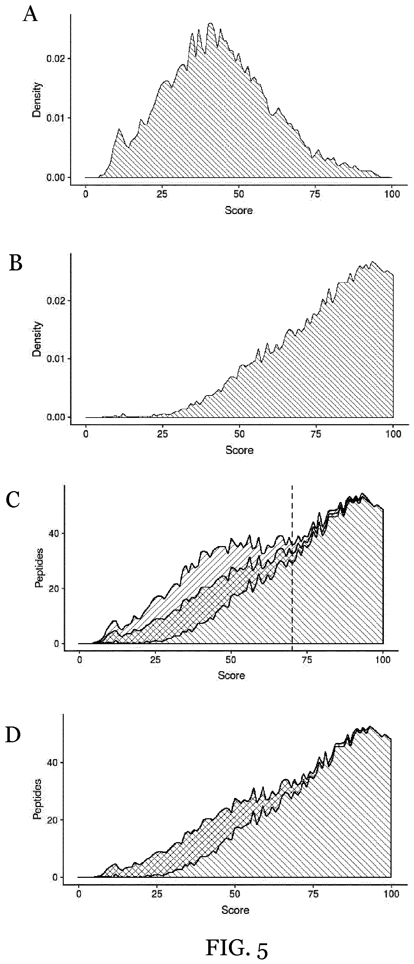

FIG. 5 shows several score distributions. Diagram A shows the score distribution of false peptides *p_{false}*(*x*), and diagram B the score distribution of true peptides *pₜᵣᵤₑ*(*x*). Diagram C shows the score histogram of 500 false targets and 2000 true targets. False targets are stacked on top of the true targets. Diagram D shows the score histogram of 500 decoys, 500 false targets and 2000 true targets. False targets and decoys are stacked on top of the true targets. The 1% FDR threshold is indicated.

Classes 1 und 2 have the same score distribution with density *p_{false}*(*x*), and class 3 has a distinct score distribution with density *pₜᵣᵤₑ*(*x*) (see diagrams A and B in FIG. 5). We cannot observe class 2 and 3 separately, but the mixture of both (i.e. the scores of the target database; see diagram C in FIG. 5). However, we can observe the score histogram of decoys, which is the same as for false targets based on the master condition (see diagram D in FIG. 5). For any threshold *t,* the number of decoys exceeding *t* equals the number of false targets exceeding *t.* Thus, we can choose a threshold such that the percentage of false targets is 1% of all targets exceeding *t* (see diagram D in FIG. 5).

### 2. TDA for de novo peptide sequencing

As defined above, the third component for TDA is a program that, based on an input database D, assigns a peptide from D and an associated score to each tandem mass spectrum. Usually this program is a sequence database search tool such as Mascot, Sequest, Andromeda or X!Tandem. However, given any de novo peptide sequencing tool (such as implemented in Peaks), the following pseudocode also constitutes such a program:
1. Perform de novo peptide sequencing
2. Output the top n candidates for each spectrum
3. Find the top-most candidate that occurs in the sequence database

Such an approach has been proposed earlier. There, the fundamental problem was to identify the "*n*" for a given spectrum, i.e. how many candidates must be considered to identify one that occurs in the sequence database. We noticed that due to (i) high mass accuracy tandem mass spectra and (ii) the length restriction (8-11 amino acids) of MHC-I peptides, the top candidate is correct for a large fraction of spectra and among the top 10 for virtually all spectra where otherwise a peptide could be identified by standard database search.

### 3. Stratified databases

The theory behind TDA also applies if the target database actually consists of multiple strata, where each stratum has a different size and likelihood of identifying a peptide. However, the estimated FDR is global and does not allow drawing conclusions for individual strata.

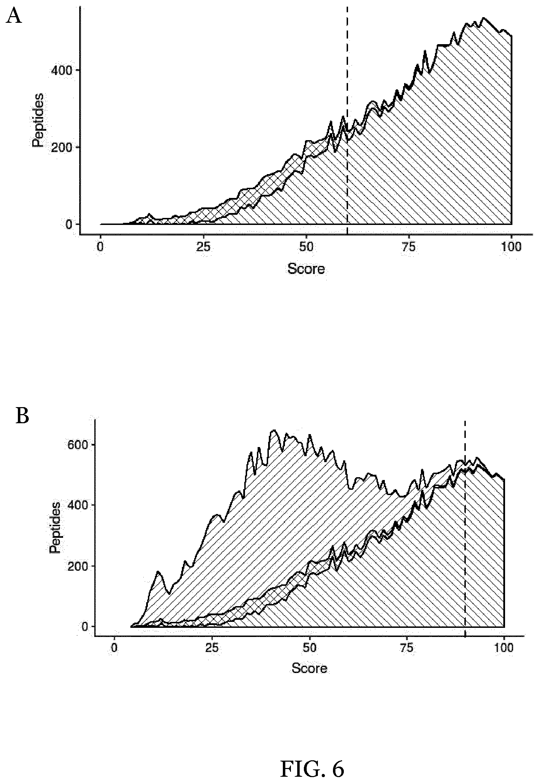

Example: The target database consists of strata A and B. 9900 peptides from A and 100 peptides from B have been found at a global FDR of 1%. Clearly, we expect 100 peptides to be false discoveries. The TDA does not provide arguments to assume a 1% FDR for both A and B. Thus, all peptides from B could be false. This case can indeed happen, e.g. if B completely consists of false peptides, and is large enough (see FIG. 6).

FIG. 6 shows score distributions for stratified databases. Diagram A of FIG. 6 shows the distribution of false and true targets in the database stratum A from the example (see Text). The 1% FDR threshold is indicated. Diagram B shows the same as diagram A, but with the targets from stratum B (that are all false) added. The now shifted 1% FDR threshold is indicated.

A straightforward solution is to employ TDA per stratum. However, as individual strata can be small, which would lead to highly imprecise FDR estimates, we follow a different path in Peptide-PRISM (this is done for each peptide length l): We use the histogram of all decoy peptides to estimate the score distribution of false hits with density *pₗ*(*x*) (by using a non-parametric fit based on a uni-modal regression model). Then, based on the most promising stratum (that has the greatest number of true hits, i.e. CDS), we estimate the score distribution with density *pₗ*(*x*) of true hits (by first subtracting the CDS decoy histogram from the CDS target histogram, and then applying the same fitting procedure as for decoys). We assume that for any stratum/category c, the actual score distribution of targets is a mixture distribution of these two components with density *f_{l,c}*(*x*) = *w_{l,c}* · *pₗ*(*x*) + (1 - *w_{l,c}*) · *nₗ*(*x*)*.* Here, *w_{l,c}* is the total fraction of true targets of c with length l and can be used to estimate the total number of true targets from c. In Peptide-PRISM, *w_{l,c}* is estimated numerically using maximum likelihood. Then, the mixture distribution is fully specified and allows computing FDRs (and other statistics).

### 4. Overall model design

The mixture model for all peptides of length l consists of two component distributions: One for false hits, and one for true hits. It is important to emphasize that we estimate a single distribution of false hits and a single distribution of true hits for each length l. This is based on our observation that the score distribution of decoys originating from CDS sequences is not different from the distribution of decoys from any other stratum (Peptide-PRISM generates diagnostic plots in each run to check this). This means that the potentially distinct amino acid composition in different strata has no significant effect on the score distribution. Consequently, we also use a single distribution for true hits.

Thus, for each stratum, the observed score distribution of targets (for peptides of length l) is a mixture of these two distributions, and the only difference is the relative contribution of the two mixture components, respectively (e.g. for the CDS stratum, the true hit distribution generally has more weight than for the intergenic stratum). These contributions can be estimated using maximum likelihood, and FDRs can be directly extracted from the mixture model.

### 5. Estimating the false hit distribution

For estimating the false hit distribution, we use all decoy hits of length l from all strata. As pointed out above, there is no significant difference for decoys generated from different strata. Thus, we can use all available decoys to obtain estimates as precise as possible.

### 6. Estimating the true hit distribution

Decoys are by definition false hits. For true hits, it is not possible to generate such a database. As Peptide-PRISM always generates decoys, we use a more straight-forward way to estimate the true hit distribution: In any stratum and for any peptide length, we have *dₛ* hits in the decoy database and *tₛ* hits in the target database for some (integer) identification score s. Extending the argument of the target decoy approach, the expected number of (false) decoy hits with score s is the same as the expected number of false target hits with score s. Thus, we can obtain the expected number of true target hits with score s as *tₛ* - *dₛ.* In Peptide-PRISM, we always use only use the histogram from the most promising stratum, i.e. the one that induces the highest total number of expected true targets.

### 7. Non-parametric fit

In principle, we could use the true hit and false hit histograms directly in place of the two component distributions of the mixture model. However, due to the limited sample size, both are, to some extent, noisy (again, this can be inspected in the diagnostic plots produced by Peptide-PRISM). To smooth these rugged histograms, we tested several alternatives and obtained the visually best results using a non-parametric method (uni-modal regression). This is based on much weaker assumptions than the Peptide Prophet, which fits a gamma distribution for the false hit distribution, and a normal distribution for the true hit distribution. It alternatively allows using kernel density estimates as a semi-parametric method, which is not appropriate for fitting the ALC scores produced by the de-novo search of Peaks, as they are bounded between 0 and 100, which leads to considerable boundary artifacts when kernel density estimators are used.

### 8. Overlap of target and decoy sequences

For the standard TDA, peptides that are both in the target and the decoy database pose a problem: They could be true hits, but if they are treated as targets, the decoy database is effectively smaller than the target database. This can be handled by using a corresponding factor when computing the FDR. For our mixture modeling approach, overlaps of target and decoy sequences do not represent such a problem: The decoys are only used to construct the false hit score distributions. FDRs are based on the mixture model that is estimated from targets alone (i.e. we can safely treat all ambiguous peptides as targets).

## Claims

1. A method for determining MHC class I-presented peptides from fragment ion mass spectra, the method comprising:
- de novo peptide sequencing (110) from the fragment ion mass spectra to generate candidate peptide sequences;
- determining (130), in a database, matching peptide sequences which match said candidate peptide sequences, wherein the database comprises target peptide sequences and decoy sequences;
- classifying the matching peptide sequences into classes of peptides, wherein each class refers to a functionally confined part of the genome;
- for each peptide length:
estimating a probability distribution of de novo peptide sequencing scores for false hits based on decoy hits, and estimating a probability distribution of true hits based on subtracting of decoy hits from target hits, and
for each class: determining mixture coefficients of a mixture model of the true and false probability distributions; and
- filtering (160) the matching peptide sequences using a statistical measure determined based on the mixture model to determine the MHC class I-presented peptides.

2. The method of claim 1, wherein the fragment ion mass spectra originate from a sample of a subject and the target peptide sequences are amino acid sequences of the species of the subject,
wherein in particular the target peptide sequences comprise peptide sequences corresponding to the 6-frame translated genome and/or 3-frame translated transcriptome.

3. The method of claim 1 or 2, wherein
- the target sequences comprise variant target sequences, such as generated due to mutations from DNA sequences, proteasome-catalyzed peptide splicing, all possible single amino acid substitutions (from annotated proteins) and/or frameshift peptides and/or
- the decoy sequences comprise inverted sequences.

4. The method of one of the previous claims, wherein the database matching comprises using the Aho-Corasick algorithm to scan said target sequences and optionally said variants of said target sequences, in addition to scanning all considered sequences in their inverted form.

5. The method of one of the previous claims, wherein classifying of the matching peptide sequences comprises prioritizing and/or filtering hits and/or reducing to unique sequences.

6. The method of claim 5, wherein the prioritization is performed by successively searching within all sequences belonging to two or more of the classes CDS, 5'-UTR, off-frame, 3'-UTR, ncRNA, intronic, and intergenic.

7. The method of one of the previous claims, wherein the fragment ion mass spectra are from a subject sample, which is derived from a patient, in particular a cancer patient.

8. The method of one of the previous claims, wherein the top n sequence candidates of each fragment ion spectrum are used for database matching, wherein n is an integer between 5 and 200, preferably between 7 and 20.

9. The method of one of the previous claims, wherein determining the matching peptide sequences comprises adding isobaric amino acid substitutions (L -> I), the amino acid variants N -> D within the sequence motif NG, and the amino acid variants Q -> E within sequence candidates with N-terminal pyro-glutamate to the Aho-Corasick keyword trie for all candidate sequences.

10. The method of any one of claims 1 to 5, wherein the classes comprise at least one of:
CDS (in-frame with annotated protein),
5'-UTR (contained in annotated mRNA, consistent with its introns, overlapping with 5'-UTR),
off-frame (off-frame contained in the coding sequence, consistent with its introns),
3'-UTR (all others that are intron-consistently contained in an mRNA),
ncRNA (intron-consistently contained in an annotated ncRNA),
intronic (intersecting any annotated intron),
or
intergenic.

11. The method of one of the previous claims, wherein the fragment ion mass spectra have been obtained using collision-induced dissociation (CID), higher-energy C-trap dissociation (HCD), electron transfer dissociation (ETD) or combinations of these fragmentation techniques.

12. A device for determining MHC class I-presented peptides from fragment ion mass spectra, wherein the device is configured to carry out the method of one of claims 1 to 11.

13. A computer-readable storage medium storing program code, the program code comprising instructions that when executed by a processor carry out the method of one of claims 1 to 11.
